# EUROPEAN PATENT SPECIFICATION

(11) **EP 4 179 988 B1**
(45) Date of publication and mention of the grant of the patent: **05.02.2025**
(21) Application number: 21837090.6
(22) Date of filing: 24.06.2021
(51) Int. Cl.: A61B 18/12, A61B 18/14

(54) **SURGICAL OPERATING SYSTEM AND UNIPOLAR AND BIPOLAR MIXED OUTPUT METHOD THEREFOR**
CHIRURGISCHES BETRIEBSSYSTEM UND UNIPOLARES UND BIPOLARES GEMISCHTES AUSGANGSVERFAHREN DAFÜR
SYSTÈME D'ACTIONNEMENT CHIRURGICAL ET PROCÉDÉ DE SORTIE MIXTE UNIPOLAIRE ET BIPOLAIRE ASSOCIÉ

(30) Priority: 08.07.2020 CN 202010652994; 10.07.2020 CN 202010662241
(43) Date of publication of application: 17.05.2023
(73) Proprietor: Simai Co., Ltd., 519000 Zhuhai Guangdong (CN)
(72) Inventor: LIU, Chunxiao, Zhuhai, Guangdong 519000 (CN); LIN, Min, Zhuhai, Guangdong 519000 (CN); LUO, Weitao, Zhuhai, Guangdong 519000 (CN); LI, Pengfei, Zhuhai, Guangdong 519000 (CN)
(74) Representative: Zaboliene, Reda
(86) International application number: PCT/CN2021/102016
(87) International publication number: WO 2022/007636

(56) References cited:
- EP-A1- 2 974 682
- EP-A1- 3 158 963
- CN-A- 105 877 836
- CN-A- 105 877 836
- CN-A- 107 468 340
- CN-A- 110 381 870
- CN-A- 111 671 515
- CN-U- 208 481 456
- US-A1- 2007 203 482
- US-A1- 2008 287 948
- US-B1- 7 094 231

## Description

### TECHNICAL FIELD

The present disclosure relates to the technical field of electrosurgeries, more particularly to a surgical operating system and a monopolar-bipolar hybrid output method applied to the system.

### BACKGROUND

Electrosurgical knife is employed to complete cutting and blood coagulation of human tissues using the energy output by an electrosurgical system. The thermal effect of current will cause vaporization of human tissues, which is the principle of cutting; the thermal effect of current will cause denaturation and coagulation of proteins in human tissues, which is the principle of blood coagulation.

At present, the electrosurgical system generally has a monopolar output function and a bipolar output function.

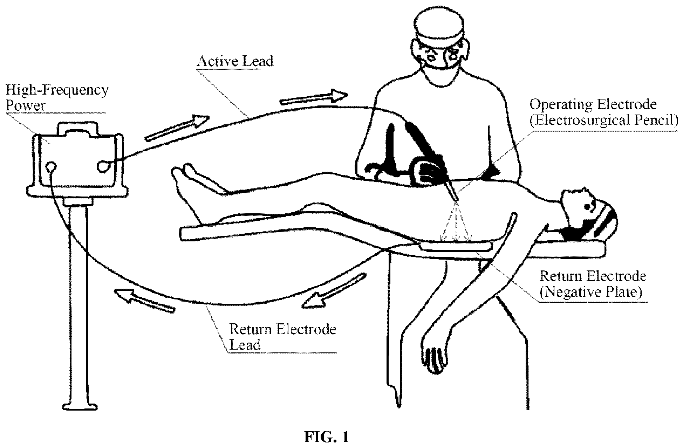

In existing technologies, as shown in FIG. 1, which is a work mode of a monopolar output function of an electrosurgical system in existing technologies, the electrosurgical system includes a high-frequency power module, an electrosurgical pencil, a return electrode, an active lead and a return electrode lead; the specific workflow of the work mode of the monopolar output function of the electrosurgical system is that the current flows from the electrosurgical pencil to the negative plate attached onto a patient body via human tissues to form a loop.

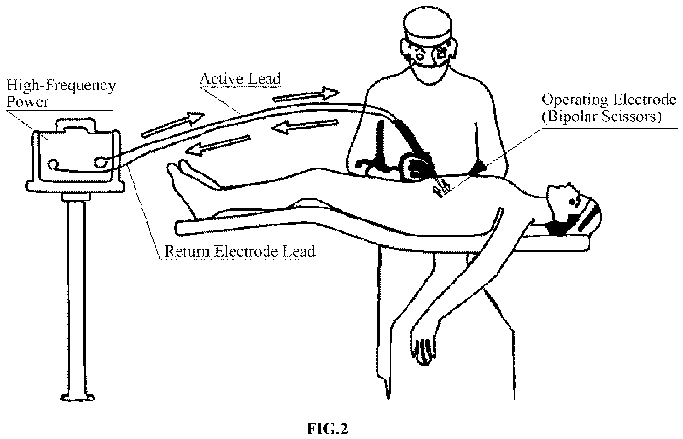

As shown in FIG. 2, which is a work mode of a bipolar output function of an electrosurgical system in existing technologies, the electrosurgical system includes bipolar scissors, an active lead and a return electrode lead; the specific workflow of the work mode of the bipolar output function of the electrosurgical system is that a bipolar instrument grips tissues between bipolar scissors (or bipolar tweezers, bipolar forceps), so that the current flows through human tissues to complete vaporization and coagulation hemostasis of the tissues.

Therefore, existing electrosurgical systems generally have monopolar output and bipolar output functions. The monopolar output has advantages that the monopolar electrosurgical knife of a monopolar output mode has a better cutting effect compared to the bipolar electrosurgical knife since the monopolar pencil tip is of greater energy density.

However, since the electrosurgical pencil is one electrode of the whole current loop, the electric power cannot be greatly concentrated onto the tissue to be coagulated, but flows through the pencil tip and then through a large area of human tissues to reach the negative plate end. The blood coagulation effect is worse than that of the bipolar electrosurgical knife; in addition, the monopolar electrosurgical pencil is difficult to reach deep into the abdominal cavity to perform laparoscopic minimal invasive surgeries; the monopolar electrosurgical pencil cannot have the aid of a mechanical shear force when cutting, and thus needs a higher power to cut open the tissues; however, the higher power will bring greater tissue thermal injuring.

The bipolar output has the following advantages. The tissue to be coagulated is gripped between the two electrodes when being coagulated, so that the electric power is greatly concentrated onto the tissues between the two electrodes. The bipolar scissors (or bipolar tweezers, bipolar forceps) have better blood coagulation effects than the monopolar electrosurgical knife, and the bipolar mechanical structure feature enables a shearing force during cutting which the monopolar electrosurgical knife does not have.

However, since the bipolar instrument (bipolar scissors or bipolar tweezers, bipolar forceps) itself generally has a smaller energy output than the monopolar instrument, and the tissue between the upper blade and the lower blade has a smaller energy density than at the tip position of the monopolar electrosurgical pencil, the bipolar instrument is difficult to achieve rapid vaporization and separation of tissues when cutting thick tissues. Therefore, the bipolar instrument (bipolar scissors or bipolar tweezers, bipolar forceps) generally can only cut thin tissues.

To sum up, the monopolar output mode is more conducive to cutting, but not conducive to blood coagulation; the bipolar output mode is more conducive to blood coagulation, but not conducive to cutting.

US7094231B1 discloses a dual-mode electrosurgical instrument.

EP2974682A1 and EP3158963 both disclose a combination electrosurgical device.

US20080287948A1 discloses an electrosurgical system.

CN105877836A discloses a surgery energy platform system.

### SUMMARY

The present invention is defined by appended claim 1. Methods do not form part of the claimed scope.

It is the main object of the present disclosure to provide a monopolar-bipolar hybrid output method which may enable monopolar-bipolar hybrid output on one same operating instrument.

It is another object of the present disclosure to provide a surgical operating system which may enable monopolar-bipolar hybrid output on one same operating instrument.

In order to achieve the above objects, a monopolar-bipolar hybrid output method provided by the present disclosure, applied to the surgical operating system, includes: determining an instruction selected by an operator for an output mode, and when obtaining that an instruction for a monopolar-bipolar hybrid mode is selected, controlling a first operating instrument to perform cutting and tissue coagulation actions.

Further, in the monopolar-bipolar hybrid mode, when performing the cutting action, a master control unit enables, through a first set of control signals, the current to flow through a first operating end of the first operating instrument and then through human tissues to reach a return electrode to form a first current loop and complete the cutting action in the monopolar-bipolar hybrid mode on the first operating instrument.

Further, in the monopolar-bipolar hybrid mode, when performing the tissue coagulation action, the master control unit enables, through a second set of control signals, the current to flow through the first operating end of the first operating instrument and then through the tissue to be cut off to reach a second operating end of the first operating instrument to form a second current loop and complete the tissue coagulation action in the monopolar-bipolar hybrid mode on the first operating instrument.

Further, the output mode further includes a monopolar mode, in which mode the master control unit enables, through a third set of control signals, the current to flow through a third operating end of a second operating instrument and then through human tissues to reach the return electrode to form a third current loop and complete the cutting action and the tissue coagulation action in the monopolar mode on the second operating instrument.

Further, the output mode further includes a bipolar mode, in which mode the master control unit enables, through a fourth set of control signals, the current to flow through the first operating end of the first operating instrument and then through human tissues to reach the second operating end of the first operating instrument to form a fourth current loop and complete the cutting action and the tissue coagulation action in the bipolar mode on the first operating instrument.

In order to achieve the another object above, the present disclosure provides a surgical operating system, including: a master control unit, a control panel (1) and an operating instrument, wherein the master control unit includes a central control unit (2), a high-frequency power module (3) and a switch matrix (4), the high-frequency power module (3) is connected to the central control unit (2) and the switch matrix (4) respectively, the central control unit (2) is configured to receive a selected instruction output by the control panel (1) and read mode information of an inbuilt chip of the operating instrument or a footswitch instruction, and the central control unit (2) is configured to output a switch drive signal to the switch matrix (4) to control the switch matrix (4) to switch on and drive the operating instrument.

Further, the switch matrix (4) comprises a first switch tube (41), a second switch tube (42), a third switch tube (43) and a fourth switch tube (44); the central control unit (2) is configured to send a switch drive signal to the first switch tube (41), the second switch tube (42), the third switch tube (43) and the fourth switch tube (44) according to the received selected instruction, to control the connection and disconnection of the first switch tube (41), the second switch tube (42), the third switch tube (43) and the fourth switch tube (44).

Further, the switch matrix (4) further includes a sixth switch tube and a seventh switch tube; the central control unit (2) is configured to send a switch drive signal to the sixth switch tube and the seventh switch tube according to the received selected instruction, to control the connection and disconnection of the sixth switch tube and the seventh switch tube.

Further, the operating instrument includes a first operating instrument, a second operating instrument and a return electrode (5); a first output socket (21) of the switch matrix (4) is connected to a tail end of the first operating instrument, a second output socket (22) of the switch matrix (4) is connected to a tail end of the second operating instrument and a tail end of the return electrode respectively(5), wherein the first operating instrument is a bipolar instrument (7), and the second operating instrument is an electrode structure (6).

Further, the bipolar instrument (7) includes a first conductive region (73), a second conductive region (74), a handle assembly (75) and a bipolar instrument operating end; the first conductive region (73) is connected to a first output end of the first output socket (21), the second conductive region (74) is connected to a second output end of the first output socket (21), and the handle assembly (75) is connected to the bipolar instrument operating end, wherein the bipolar instrument operating end includes a first operating end (71) and a second operating end (72), and the electrode structure (6) includes a third operating end.

The present disclosure has beneficial effects as follows. The one same operating instrument in the present disclosure may work in a monopolar-bipolar hybrid output mode; in the monopolar-bipolar hybrid output mode, a hybrid mode of monopolar cutting and bipolar hemostasis may be enabled on one same operating instrument, which not only overcomes respective shortcomings of conventional monopolar output and conventional bipolar output, but also integrates respective advantages of monopolar output and bipolar output.

In the monopolar-bipolar hybrid output mode, the bipolar instrument may enable the cutting of the monopolar mode, so that one operating end of the bipolar instrument is of high energy density; in addition, the bipolar instrument may complete the cutting function at low power by means of the shearing force of the bipolar instrument, causing much less injuring to the patient during the operation.

In the monopolar-bipolar hybrid output mode, the essence of blood coagulation of the bipolar instrument is still bipolar coagulation; compared to the blood coagulation effect of the monopolar mode, the bipolar coagulation has the advantage that the power is more concentrated within certain depth of the blood coagulation site and the coagulation zone is of greater depth to achieve a better hemostatic effect on the bleeding site.

According to the invention the system has the three operation modes above at the same time, being capable of switching between the output modes arbitrarily as needed in combination with the operating instrument.

### BRIEF DESCRIPTION OF THE DRAWINGS

FIG. 1 is a diagram of a work mode of a monopolar output function of an electrosurgical system in existing technologies.
FIG. 2 is a diagram of a work mode of a bipolar output function of an electrosurgical system in existing technologies.
FIG. 3 is a functional block diagram of an embodiment of a surgical operating system according to the present disclosure.
FIG. 4 is a circuit diagram of an embodiment of a surgical operating system according to the present disclosure.
FIG. 5 is a circuit diagram of a switch matrix in an embodiment of a surgical operating system according to the present disclosure.
FIG. 6 is a structural diagram of a bipolar instrument in an embodiment of a surgical operating system according to the present disclosure.
FIG. 7 is a diagram of selection modes of a control panel in an embodiment of a surgical operating system according to the present disclosure.

The present disclosure is described below in further detail in conjunction with the drawings and embodiments.

### DETAILED DESCRIPTION OF THE EMBODIMENTS

### Embodiments of a surgical operating system:

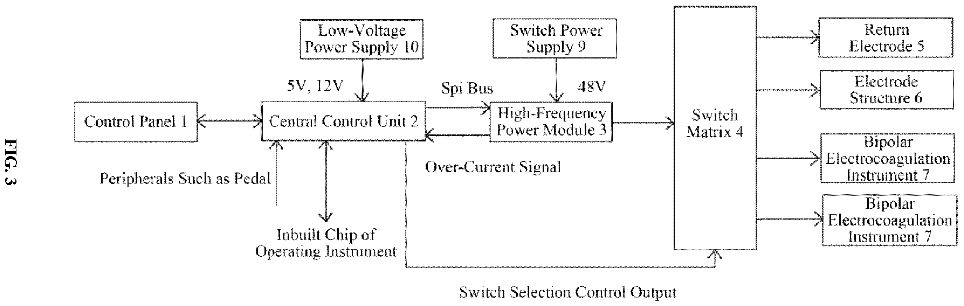

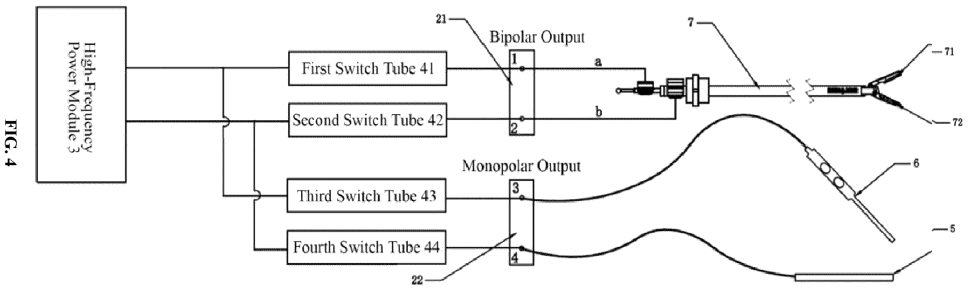

Referring to FIG. 3 and FIG. 4, the surgical operating system according to the present disclosure includes a master control unit consisting of a central control unit 2, a high-frequency power module 3 and a switch matrix 4, a control panel 1 and an operating instrument; the high-frequency power module 3 is connected to the central control unit 2 and the switch matrix 4 respectively, the central control unit 2 is configured to receive a selected instruction output by the control panel 1 and read mode information of an inbuilt chip of the operating instrument or a footswitch instruction, and the central control unit 2 is configured to output a switch drive signal to the switch matrix 4 to control the switch matrix 4 to switch on and drive the operating instrument. The selected instruction in the present embodiment may be the instruction that the user selects via the display screen of the control panel 1, also may be the instruction information that the system identifies through the electrode inbuilt chip.

Here, the central control unit 2 in the present embodiment may be chips with computing functions, such as MCU, but not limited to MCU, and it also may be other micro controllers, such as DSP, microcomputers, Complex Programmable Logic Devices (CPLD), FPGA, application specific integrated circuits and other programmable circuits.

In the present embodiment, the surgical operating system further includes a low-voltage power supply 10 and a switch power supply 9; the low-voltage power supply 10 is configured to supply 5V/12V power to the central control unit 2, and the switch power supply 9 is configured to supply 48V power to the high-frequency power module 3. Of course, the low-voltage power supply 10 and the switch power supply 9 in the present embodiment may be other voltage outputs, for example, the switch power supply may be a 24V power output.

In the present embodiment, the switch matrix 4 includes a first switch tube 41, a second switch tube 42, a third switch tube 43, a fourth switch tube 44, a first output socket 21, a second output socket 22 and at least one third output socket; the central control unit 2 is configured to send a switch drive signal to the first switch tube 41, the second switch tube 42, the third switch tube 43 and the fourth switch tube 44 according to the received selected instruction, to control the connection and disconnection of the first switch tube 41, the second switch tube 42, the third switch tube 43 and the fourth switch tube 44.

Here, the switch matrix 4 further includes a sixth switch tube and a seventh switch tube; the central control unit 2 is configured to send a switch drive signal to the sixth switch tube and the seventh switch tube according to the received selected instruction, to control the connection and disconnection of the sixth switch tube and the seventh switch tube.

Further, the first switch tube 41 and the second switch tube 42 are connected to a first output end and a second output end of the first output socket 21 respectively, the third switch tube 43 and the fourth switch tube 44 are connected to a first output end and a second output end of the second output socket 22 respectively, the sixth switch tube and the seventh switch tube are connected to a first output end and a second output end of the third output socket respectively.

The switch matrix 4 in the present embodiment preferably is a relay switch matrix, but not limited to relays, and it also may be photocouplers, triodes, power MOSFET, insulated gate bipolar transistors (IGBT) or any other controllable switches.

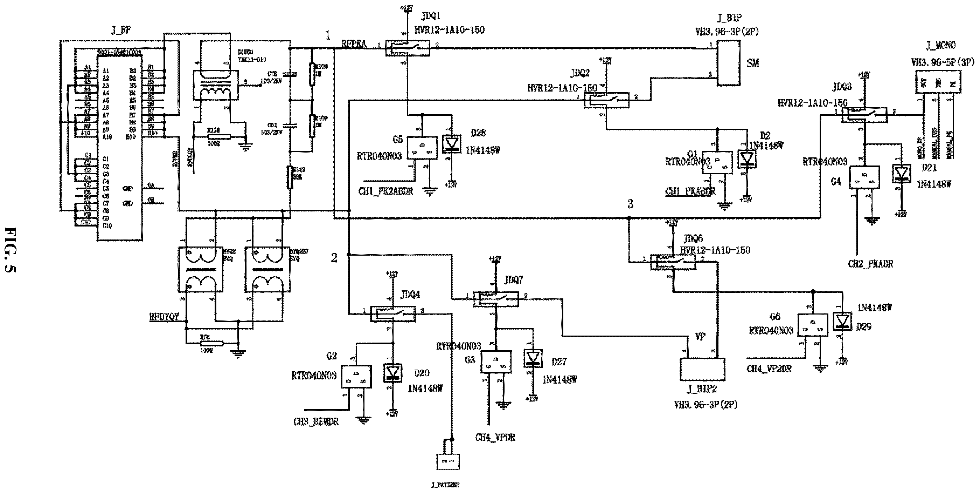

Specifically, as shown in FIG. 5, the first switch tube 41, the second switch tube 42, the third switch tube 43, the fourth switch tube 44, the sixth switch tube and the seventh switch tube are a first relay switch JDQ1, a second relay switch JDQ2, a third relay switch JDQ3, a fourth relay switch JDQ4, a sixth relay switch JDQ6 and a seventh relay switch JDQ7 respectively; the central control unit 2 switches on six relay switch drive signals through similar phase inverters (for example, 74HC14D, etc.), that is, a CH1_PK2ABDR signal input to a field effect transistor G5, a CH1_PKABDR signal input to a field effect transistor G1, a CH2_PKADR signal input to a field effect transistor G4, a CH3_BEMDR signal input to a field effect transistor G2, a CH4_VP2DR signal input to a field effect transistor G6, and a CH4_VPDR signal input to a field effect transistor G3 respectively.

Of course, the field effect transistor in the present embodiment also may be photocouplers, for example, PC817, or may be analogue switches, for example, 74HC4066, or may be triodes, etc. to complete the switch control of the relay.

Specifically, the central control unit 2 outputs a CH1_PK2ABDR signal to the grid of the field effect transistor G5, the drain of the field effect transistor G5 is connected to a third end of the first relay switch JDQ1, the central control unit 2 outputs a CH1_PKABDR signal to the grid of the field effect transistor G1, the drain of the field effect transistor G1 is connected to a third end of the second relay switch JDQ2, the central control unit 2 outputs a CH2_PKADR signal to the grid of the field effect transistor G4, the drain of the field effect transistor G4 is connected to a third end of the third relay switch JDQ3, the central control unit 2 outputs a CH3_BEMD signal to the grid of the field effect transistor G2, the drain of the field effect transistor G2 is connected to a third end of the fourth relay switch JDQ4, the central control unit 2 outputs a CH4_VP2DR signal to the grid of the field effect transistor G6, the drain of the field effect transistor G6 is connected to a third end of the sixth relay switch JDQ6, the central control unit 2 outputs a CH4_VPDR signal to the grid of the field effect transistor G3, the drain of the field effect transistor G3 is connected to a third end of the seventh relay switch JDQ7. The sources of the field effect transistor G1, the field effect transistor G2, the field effect transistor G3, the field effect transistor G4, the field effect transistor G5, the field effect transistor G6 and the field effect transistor G7 are grounded respectively; a first end of the first relay switch JDQ1 is connected to a node 1, the node 1 is connected to a node 3, a first end of the third relay switch JDQ3 and a first end of the sixth relay switch JDQ6 are connected to the node 3. A first end of the second relay switch JDQ2, a first end of the fourth relay switch JDQ4 and a first end of the seventh relay switch JDQ7 are connected to the node 2. A second end of the first relay switch JDQ1 and a second end of the second relay switch JDQ2 are connected to the first output socket 21 (for example, SM output socket), a second end of the sixth relay switch JDQ6 and a second end of the seventh relay switch JDQ7 are connected to a third output socket (for example, VP output socket), a second end of the third relay switch JDQ3 and a second end of the fourth relay switch JDQ4 are connected to the second output socket 22 (for example, J_MONO and J_PATIENT).

In the present embodiment, the operating instrument includes a first operating instrument, a second operating instrument and a return electrode 5; the first output socket 21 of the switch matrix 4 is connected to a tail end of the first operating instrument, a second output socket 22 of the switch matrix 4 is connected to a tail end of the second operating instrument and a tail end of the return electrode 5 respectively, wherein the first operating instrument is a bipolar instrument 7, the second operating instrument is an electrode structure 6, and the return electrode 5 is a negative plate (abdominal board). In the present embodiment, the second output socket 22 may be electrically divided into two interface sockets, that is, one interface socket is connected to the port 3 of the second output socket 22 while the other interface socket is connected to the port 4 of the second output socket 22, one interface socket is connected to the second operating instrument while the other interface socket is connected to the return electrode 5.

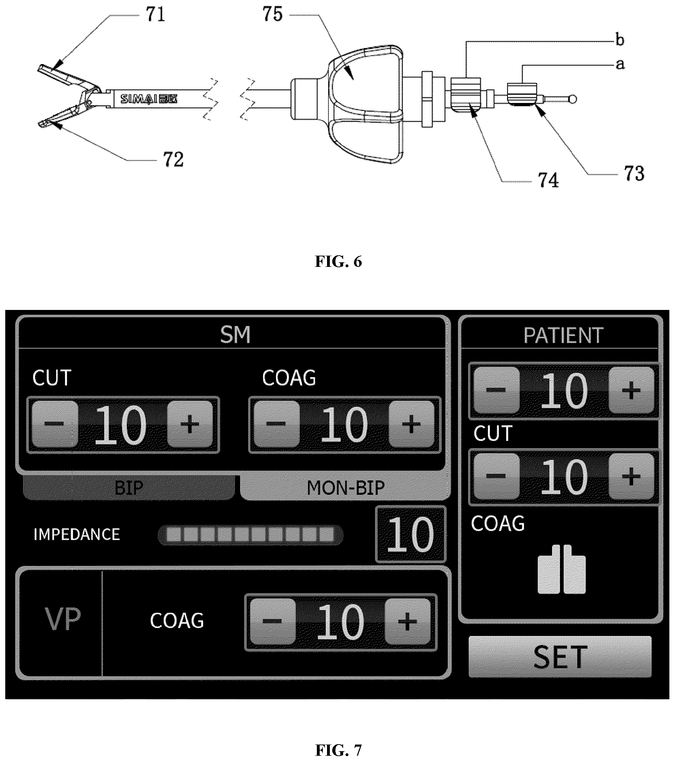

Referring to FIG. 6, the bipolar instrument 7 includes a first conductive region 73, a second conductive region 74, a handle assembly 75 and a bipolar instrument operating end; the first conductive region 73 is connected to the first output end of the first output socket 21, the second conductive region 74 is connected to the second output end of the first output socket 21 (or vice versa), and the handle assembly 75 is connected to the bipolar instrument operating end. The bipolar instrument 7 in the present embodiment preferably is bipolar electrocoagulation scissors, but not limited thereto, and it also may be bipolar electrocoagulation forceps, bipolar electrocoagulation tweezers and other bipolar electrocoagulation devices. The electrode structure 6 in the present embodiment preferably is a monopolar electrosurgical pencil module or similar structures.

The bipolar instrument operating end includes a first operating end 71 and a second operating end 72, and the electrode structure 6 includes a third operating end; the first conductive region is electrically connected to the first operating end of the bipolar operating instrument, and the second conductive region is electrically connected to the second operating end of the bipolar operating instrument; or, the first conductive region is electrically connected to the second operating end, and the second conductive region is electrically connected to the first operating end. Here, the first operating end 71 of the bipolar instrument 7 is an upper blade end, the second operating end of the bipolar instrument 7 is a lower blade end, the third operating end of the electrode structure 6 is a knife head end.

The high-frequency power module 3 in the present embodiment has three energy output modes, which are corresponding to three output modes of the surgical operating system of the present disclosure respectively, namely a monopolar output mode, a bipolar output mode and a monopolar-bipolar hybrid output mode. The operating system works in the monopolar output mode, the bipolar output mode or the monopolar-bipolar hybrid output mode through the master control unit. When the monopolar-bipolar hybrid output mode is selected, the operating system may enable monopolar-bipolar hybrid output on one same operating instrument.

When the monopolar-bipolar hybrid output mode is selected, during the operation of cutting, the current flows through the blade end of the bipolar instrument 7 to the negative plate via human tissues (the essence of which is monopolar cutting); during the operation of blood coagulation, the current flows through the upper blade end of the bipolar instrument 7 and then through the human tissues gripped between the bipolar instrument 7 to the lower blade end of the bipolar instrument 7 (the essence of which is bipolar coagulation). Therefore, monopolar-bipolar hybrid output (monopolar cutting, bipolar coagulation) and bipolar output (bipolar cutting, bipolar coagulation) may be enabled on one operating instrument.

Specifically, when the bipolar instrument 7 is employed to perform tissue cutting, it is the monopolar energy output mode that is used; at such time, energy is output on the upper blade of the bipolar instrument 7 only, and the current flows through the tissues to reach the negative plate; when the bipolar instrument 7 is employed to perform blood coagulation of tissues, it is the bipolar energy output mode that is used; at such time, the current flows between the upper blade end and the blower blade end of the bipolar instrument 7 and does not flow through the negative plate. That is to say, on one same operating instrument (bipolar instrument 7) are not only integrated respective advantages of monopolar and bipolar energy output modes, but also are overcome respective original shortcomings of monopolar and bipolar energy output modes.

In the monopolar mode, the return electrode 5 is electrically connected to the first output end (line 4 or line 3) of the second output socket 22, and the electrode structure 6 is electrically connected to the second output socket 22 (line 3 or line 4). When the monopolar mode is selected, the system closes the third switch tube 43 or the fourth switch tube 44 to form a monopolar loop, to complete monopolar cutting and blood coagulation.

In the bipolar mode, the bipolar instrument 7 is connected to the first output socket 21 (bipolar output socket); when the bipolar mode is selected, the system closes the first switch tube 41 or the second switch tube 42 to form a bipolar loop, to complete bipolar cutting and blood coagulation.

When the monopolar-bipolar hybrid output mode is selected, the bipolar instrument 7 is connected to the first output socket 21, and the return electrode 5 is connected to the second output socket 22.

When a cutting pedal is enabled or a cutting button is hand controlled, the system closes the first switch tube 41, connects to the fourth switch tube 44 of the return electrode 5, and disconnects to the second switch tube 42 and the third switch tube 43, so that the current flows through the operating end (blade end) of the bipolar instrument 7 and then through human tissues to reach the return electrode 5 to form a current loop, namely, forming a monopolar loop in essence, so as to complete monopolar cutting on the bipolar instrument 7.

When a coagulation pedal is enabled or a coagulation button is hand controlled, the system closes the first switch tube 41 and the second switch tube 42, disconnects to the fourth switch tube 44 of the return electrode 5, and disconnects to the third switch tube 43 of the electrode structure 6, so that the current flows through one operating end (for example, upper blade end) of the bipolar instrument 7 and then through human tissues to reach the other operating end (for example, lower blade end) of the bipolar instrument 7, namely, forming a bipolar coagulation loop in essence, so as to achieve bipolar coagulation effects.

In the present embodiment, the high-frequency power module 3 further has a fourth energy output mode, which is corresponding to a fourth output mode of the surgical operating system of the present disclosure, that is, bipolar VP mode.

The bipolar VP mode of the present disclosure is a second bipolar mode output or more than two bipolar mode outputs and has the same output principle as the bipolar mode, that is, adding the sixth switch tube and the seventh switch tube and adding a corresponding bipolar instrument 7 onto a corresponding output socket.

Preferably, the control panel 1 of the present embodiment may be a touch display screen, but not limited thereto, and it also may be a metal dome array, also may be any inductive display devices capable of receiving input signals such as contacts.

Specifically, the central control unit 2 controls the high-frequency power module 3 through an interface and a radio frequency adapter (for example, J_RF1 socket), and the high-frequency power module 3 outputs to a patient through a J_RF1 socket and a relay switch matrix. Here, the communication mode between the central control unit 2 and the high-frequency power module 3 may be a serial data or parallel data format. The serial data format may be SPI, IIC, uart, one-line bus, etc.; the parallel data format may be asynchronous clocks such as 8 bits, 16 bits, 32 bits, etc., also may be synchronous clocks such as 8 bits, 16 bits, 32 bits, etc.

Embodiments of a monopolar-bipolar hybrid output method for a surgical operating system:

Referring to FIG. 7, the monopolar-bipolar hybrid output method of the present disclosure is applied to an electrosurgical operating system, including: determining through the control panel 1 a selected instruction for the monopolar mode, the bipolar mode or the monopolar-bipolar hybrid mode, wherein the instruction is given by an operator, or is given by the mode information read by the operating instrument inbuilt chip, when the master control unit obtains that an instruction for the monopolar-bipolar hybrid mode is selected, controlling the first operating instrument to perform cutting actions and tissue coagulation actions.

Specifically, the above operating system performs information exchange with the central control unit 2 through the touch display screen. As for mode selection, when the system is turned on, the touch display screen enters a standby interface, the right area is a monopolar area, the left area is a bipolar mode area, wherein the left upper area is a bipolar SM area, and the left lower area is a bipolar VP control area.

During practical applications, the left upper bipolar SM area includes two mode selection buttons; as for a first mode selection button, when the first bipolar mode button is pressed, a bipolar output mode is selected, that is, selection is performed through the relay so that the current flows through one operating end of the bipolar instrument 7 and then through the human tissues gripped between the bipolar instrument 7 to reach the other operating end of the bipolar instrument 7; as for a second mode selection button, when the second bipolar mode button is pressed, a monopolar-bipolar hybrid mode is selected, and the system, when detecting that the return electrode 5 is connected to a patient already, will perform selection through the relay, so that during the cutting process the current flows through the operating end of the bipolar instrument 7 and then through the human tissues to reach the return electrode 5 (which is monopolar cutting in essence), and during the blood coagulation process the current flows through one operating end of the bipolar instrument 7 and then through the human tissues gripped between the bipolar instrument 7 to reach the other operating end of the bipolar instrument 7 (which is bipolar coagulation in essence).

In the present embodiment, the system may read electrode information through a bus, thereby confirming according to the read electrode information whether the default output mode of the electrode is bipolar output or monopolar-bipolar hybrid output.

In the monopolar-bipolar hybrid mode, when performing the cutting action, the master control unit enables, through a first set of control signals, the current to flow through a first operating end 71 of the first operating instrument and then through human tissues to reach the return electrode 5 to form a first current loop and complete the cutting action in the monopolar-bipolar hybrid mode on the first operating instrument.

In the present embodiment, in the monopolar-bipolar hybrid mode, when performing the tissue coagulation action, the master control unit enables, through a second set of control signals, the current to flow through the first operating end 71 of the first operating instrument and then through the tissue to be cut off to reach the second operating end 72 of the first operating instrument to form a second current loop and complete the bipolar tissue coagulation action in the monopolar-bipolar hybrid mode on the first operating instrument.

In the monopolar mode, the master control unit enables, through a third set of control signals, the current to flow through the third operating end of the second operating instrument and then through human tissues to reach the return electrode 5 to form a third current loop and complete the cutting action and the tissue coagulation action in the monopolar mode on the second operating instrument.

In the bipolar mode, the master control unit enables, through a fourth set of control signals, the current to flow through the first operating end 71 of the first operating instrument and then through human tissues to reach the second operating end 72 of the first operating instrument to form a fourth current loop and complete the cutting action and the tissue coagulation action in the bipolar mode on the first operating instrument.

Of course, the first set of control signals, the second set of control signals, the third set of control signals and the fourth set of control signals of the present embodiment are all output by the high-frequency power module 3 and the relay matrix under the control of the central control unit 2 to form various current loops.

When to use the monopolar mode, press the electrode structure 6 (for example, electrosurgical pencil cutting) or coagulation button, through the circuit is triggered an optical coupling signal of the optical coupler or an amplified electric signal of an operational amplifier, and the optical coupling signal or the amplified electric signal is transmitted to the central control unit 2, then the central control unit 2 switches on the relay switch drive signals CH2_PKADR and CH3_BEMDR through similar phase inverters, so that the fourth switch tube 44 (for example, the fourth relay switch JDQ4) and the third switch tube 43 (for example, the third relay switch JDQ3) are switched on, and the central control unit 2 controls the other relays to be disconnected, so that the high-frequency power module 3 outputs a high-frequency current, which flows through the third switch tube 43 (for example, the third relay switch JDQ3) to reach the electrode structure 6 (for example, electrosurgical pencil), then through human tissues to reach the return electrode 5, then through the fourth switch tube 44 (for example, the fourth relay switch JDQ4) to return back to the high-frequency power module 3, achieving monopolar cutting and coagulation.

When to use the bipolar mode, step down the pedal or hand control the cutting button or hand control the coagulation button, through the circuit is triggered a signal and the signal is transmitted to the central control unit 2 through a data transmission interface, then the central control unit 2 switches on the relay switch drive signals CH1_PK2ABDR and CH1_PKABDR through similar phase inverters, so that the first switch tube 41 (for example, the first relay switch JDQ1) and the second switch tube 42 (for example, the second relay switch JDQ2) are switched on, and the central control unit 2 controls the other relays to be disconnected, so that the high-frequency current flows through the first switch tube 41 (for example, the first relay switch JDQ1) to reach one operating end of the bipolar instrument 7, then flows through the pathological tissues gripped between the bipolar instrument 7 to reach the other operating end of the bipolar instrument 7, and then flows from the other operating end of the bipolar instrument 7, through the second switch tube 42 (for example, the second relay switch JDQ2) to return back to the high-frequency power module 3, achieving bipolar cutting and coagulation. Therefore, the operating instrument (for example, bipolar electrocoagulation scissors, forceps, tweezers) provided by the present disclosure has better clinical effects than conventional bipolar operating instruments.

When to use the monopolar-bipolar hybrid mode, in the case of performing cutting actions, step down the pedal or hand control the cutting button, through the circuit is triggered a signal and the signal is transmitted to the central control unit 2 through a data transmission interface, then the central control unit 2 switches on the relay switch drive signals CH1_PK2ABDR and CH3_BEMDR through similar phase inverters, so that the first switch tube 41 and the fourth switch tube 44 are switched on, and the central control unit 2 controls the other relays to be disconnected, so that the high-frequency current flows through the first switch tube 41 to reach one operating end of the bipolar instrument 7, then flows through human tissues to reach the return electrode 5, and then flows through the fourth switch tube 44 to return back to the high-frequency power module 3, achieving bipolar cutting.

In the case of performing tissue coagulation actions, step down the pedal or hand control the coagulation button, through the circuit is triggered a signal and the signal is transmitted to the central control unit 2 through a data transmission interface, then the central control unit 2 switches on the relay switch drive signals CH1_PK2ABDR and CH3_BEMDR through similar phase inverters, so that the first switch tube 41 and the fourth switch tube 44 are switched on, and the central control unit 2 controls the other relays to be disconnected, so that the high-frequency current flows through the first switch tube 41 to reach one operating end of the bipolar instrument 7, then flows through the pathological tissues gripped between the bipolar instrument 7 to reach the other operating end of the bipolar instrument 7, and then flows from the other operating end of the bipolar instrument 7, through the second switch tube 42 to return back to the high-frequency power module 3, achieving bipolar cutting and coagulation.

The present system further includes a bipolar VP mode output, for example, this mode is selected by touching the bipolar VP control area of the display screen, or the SM port output is switched to the VP port output through the conversion button in the middle of the foot switch. The bipolar VP mode is a second bipolar mode output or more than two bipolar mode outputs, that is, adding the sixth switch tube and the seventh switch tube and adding a corresponding bipolar electrocoagulation instrument onto a corresponding output socket. In the bipolar VP mode, the central control unit 2 switches on the relay switch drive signal through similar phase inverters, so that the sixth switch tube and the seventh switch tube (for example, the sixth relay switch JDQ6, the seventh relay switch JDQ7) are switched on, and the central control unit 2 controls the other relays to be disconnected; this mode has the same principle as the above bipolar mode, only adding one or more radio frequency output socket outputs (for example, a third output socket) to achieve different radio frequency output socket outputs; when one more radio frequency output socket is added, just add a corresponding bipolar electrocoagulation device.

The present disclosure has beneficial effects as follows. The one same operating instrument in the present disclosure may work in a monopolar-bipolar hybrid output mode; in the monopolar-bipolar hybrid output mode, a hybrid mode of monopolar cutting and bipolar hemostasis may be enabled on one same operating instrument, which not only overcomes respective shortcomings of conventional monopolar output and conventional bipolar output, but also integrates respective advantages of monopolar output and bipolar output.

In the monopolar-bipolar hybrid output mode, the bipolar instrument 7 may enable the cutting of the monopolar mode, so that one operating end of the bipolar instrument 7 is of high energy density; in addition, the bipolar instrument 7 may complete the cutting function at low power by means of the shearing force of the bipolar instrument 7, causing much less injuring to the patient during the operation.

In the monopolar-bipolar hybrid output mode, the essence of blood coagulation of the bipolar instrument 7 is still bipolar coagulation; compared to the blood coagulation effect of the monopolar mode, the bipolar coagulation has the advantage that the power is more concentrated within certain depth of the blood coagulation site and the coagulation zone is of greater depth to achieve a better hemostatic effect on the bleeding site.

In addition, the present disclosure has the three operation modes above at the same time, being capable of switching between the output modes arbitrarily as needed in combination with the operating instrument.

It is to be noted that the above are the preferred embodiments of the present disclosure, however, the design concept of the present disclosure is not limited thereto. Any non-substantive modifications made within the scope of claim 1 shall fall into the scope of protection of the present disclosure.

## Claims

1. A surgical operating system, comprising:
a master control unit, a control panel (1), a first operating instrument, a second operating instrument and a return electrode, wherein the master control unit comprises a central control unit (2), a high-frequency power module (3) and a switch matrix (4), the high-frequency power module (3) is connected to the central control unit (2) and the switch matrix (4) respectively, the high-frequency power module includes a monopolar output mode, a bipolar output mode and a monopolar-bipolar hybrid output mode,
the central control unit (2) is configured to receive a selected instruction output by the control panel (1) and read mode information of an inbuilt chip of the operating instrument or a footswitch instruction, and the central control unit (2) is configured to output a switch drive signal to the switch matrix (4) to control the switch matrix (4) to switch on and drive the first operating instrument, the second operating instrument and the return electrode (5) in one of the modes,
the surgical operating system works in the monopolar output mode, the bipolar output mode or the monopolar-bipolar hybrid output mode through the master control unit,
when obtaining that an instruction for the monopolar-bipolar hybrid output mode is selected, controlling the first operating instrument to perform cutting and tissue coagulation actions;
in the monopolar-bipolar hybrid output mode, when performing the cutting action, the master control unit enables, through a first set of control signals, the current to flow through a first operating end of the first operating instrument and then through human tissues to reach the return electrode to form a first current loop and complete the cutting action;
in the monopolar-bipolar hybrid output mode, when performing the tissue coagulation action, the master control unit enables, through a second set of control signals, the current to flow through the first operating end of the first operating instrument and then through the tissue to be cut off to reach a second operating end of the first operating instrument to form a second current loop and complete the tissue coagulation action;
when in the monopolar mode, the master control unit enables, through a third set of control signals, the current to flow through a third operating end of a second operating instrument and then through human tissues to reach the return electrode to form a third current loop and complete the cutting action and the tissue coagulation action;
when in the bipolar mode, the master control unit enables, through a fourth set of control signals, the current to flow through the first operating end of the first operating instrument and then through human tissues to reach the second operating end of the first operating instrument to form a fourth current loop and complete the cutting action and the tissue coagulation action.

2. The operating system according to claim 1, wherein
the switch matrix (4) comprises a first switch tube (41), a second switch tube (42), a third switch tube (43) and a fourth switch tube (44); the central control unit (2) is configured to send a switch drive signal to the first switch tube (41), the second switch tube (42), the third switch tube (43) and the fourth switch tube (44) according to the received selected instruction, to control the connection and disconnection of the first switch tube (41), the second switch tube (42), the third switch tube (43) and the fourth switch tube (44).

3. The operating system according to claim 2, wherein
the switch matrix (4) further comprises a fifth switch tube and a sixth switch tube; the central control unit (2) is configured to send a switch drive signal to the fifth switch tube and the sixth switch tube according to the received selected instruction, to control the connection and disconnection of the fifth switch tube and the sixth switch tube.

4. The operating system according to claim 1 or 2, wherein
a first output socket (21) of the switch matrix (4) is connected to a tail end of the first operating instrument, a second output socket (22) of the switch matrix (4) is connected to a tail end of the second operating instrument and a tail end of the return electrode (5) respectively, wherein the first operating instrument is a bipolar instrument (7), and the second operating instrument is an electrode structure (6).

5. The operating system according to claim 4, wherein
the bipolar instrument (7) comprises a first conductive region (73), a second conductive region (74), a handle assembly (75) and a bipolar instrument operating end; the first conductive region (73) is connected to a first output end of the first output socket (21), the second conductive region (74) is connected to a second output end of the first output socket (21), and the handle assembly (75) is connected to the bipolar instrument operating end, wherein the bipolar instrument operating end comprises a first operating end (71) and a second operating end (72), and the electrode structure (6) comprises a third operating end.

## Patentansprüche

1. Chirurgisches Betriebssystem, **dadurch gekennzeichnet, dass** es Folgende umfasst:
eine Hauptsteuereinheit, ein Bedienfeld (1), ein erstes Ausführungsinstrument, ein zweites Ausführungsinstrument und eine Schleifenelektrode (5), wobei die Hauptsteuereinheit eine zentrale Steuerung (2), ein Hochfrequenz-Leistungsmodul (3) und eine Schaltmatrix (4) aufweist, wobei das Hochfrequenz-Leistungsmodul (3) jeweils mit der zentralen Steuerung (2) und der Schaltmatrix (4) verbunden ist, wobei das Hochfrequenz-Leistungsmodul über einen monopolaren Ausgangsmodus, einen bipolaren Ausgangsmodus und einen gemischten monopolar-bipolaren Ausgangsmodus verfügt,
wobei die zentrale Steuerung (2) den vom Bedienfeld (1) ausgegebenen Auswahlbefehl empfängt, die Modusinformationen oder den Fußschalterbefehl des eingebauten Chips des Ausführungsinstruments liest und ausführt, und wobei die zentrale Steuerung (2) ein Antriebsschaltsignal an die Schaltmatrix (4) ausgibt, um die Schaltmatrix (4) zu steuern, um das erste Ausführungsinstrument, das zweite Ausführungsinstrument und die Schleifenelektrode (5) so zu steuern, dass sie in einem der Modi arbeiten,
wobei das chirurgische Betriebssystem durch die Hauptsteuerungseinheit in dem monopolaren Ausgangsmodus, dem bipolaren Ausgangsmodus und dem gemischten monopolar-bipolaren Ausgangsmodus betrieben wird,
wobei ein Schneidvorgang und ein Gewebekoagulationsvorgang durch Steuern des ersten Ausführungsinstruments ausgeführt werden, wenn ein Befehl zum Auswählen des gemischten monopolar-bipolaren Modus erhalten wird,
wobei beim Durchführen eines Schneidvorgangs im gemischten monopolar-bipolaren Modus die Hauptsteuereinheit mit einem ersten Satz von Steuersignalen bewirkt, dass der Strom durch ein erstes Ausführungsende des ersten Ausführungsinstruments und menschliches Gewebe zu der Schleifenelektrode fließt, um eine erste Stromschleife zu bilden, und wobei der Schneidvorgang im gemischten monopolar-bipolaren Modus auf dem ersten Ausführungsinstrument abschließt,
wobei beim Durchführen eines Gewebekoagulationsvorgangs im gemischten monopolar-bipolaren Modus die Hauptsteuereinheit mit einem zweiten Satz von Steuersignalen bewirkt, dass der Strom durch das erste Ausführungsende des ersten Ausführungsinstruments und das zu entfernende Gewebe zu einem zweiten Ausführungsende des ersten Ausführungsinstruments fließt, um eine zweite Stromschleife zu bilden, und wobei der Gewebekoagulationsvorgang im gemischten monopolar-bipolaren Modus auf dem ersten Ausführungsinstrument abschließt,
wobei im monopolaren Modus die Hauptsteuereinheit mit einem dritten Satz von Steuersignalen bewirkt, dass der Strom durch ein drittes Ausführungsende des zweiten Ausführungsinstruments und das menschliche Gewebe zu der Schleifenelektrode fließt, um eine dritte Stromschleife zu bilden, und wobei der Schneidvorgang und der
Gewebekoagulationsvorgang im monopolaren Modus auf dem zweiten Ausführungsinstrument abschließen,
wobei im bipolaren Modus die Hauptsteuereinheit mit einem vieren Satz von Steuersignalen bewirkt, dass der Strom durch das erste Ausführungsende des ersten Ausführungsinstruments und das menschliche Gewebe zu dem zweiten Ausführungsende des ersten Ausführungsinstruments fließt, um eine vierte Stromschleife zu bilden, und wobei der Schneidvorgang und der Gewebekoagulationsvorgang im bipolaren Modus auf dem ersten Ausführungsinstrument abschließen.

2. Betriebssystem nach Anspruch 1, **dadurch gekennzeichnet, dass**:
die Schaltmatrix (4) einen ersten Schaltertransistor (41), einen zweiten Schaltertransistor (42), einen dritten Schaltertransistor (43) und einen vierten Schaltertransistor (44) umfasst, wobei die zentrale Steuerung (2) Ansteuerschaltsignale an den ersten Schaltertransistor (41), den zweiten Schaltertransistor (42), den dritten Schaltertransistor (43) und den vierten Schaltertransistor (44) gemäß dem empfangenen Auswahlbefehl sendet, um das Ein- und Ausschalten des ersten Schaltertransistors (41), des zweiten Schaltertransistors (42), des dritten Schaltertransistors (43) und des vierten Schaltertransistors (44) zu steuern.

3. Betriebssystem nach Anspruch 2, **dadurch gekennzeichnet, dass**:
die Schaltmatrix (4) ferner einen fünften Schaltertransistor und einen sechsten Schaltertransistor umfasst, wobei die zentrale Steuerung (2) Ansteuerschaltsignale an den fünften Schaltertransistor und den sechsten Schaltertransistor gemäß dem empfangenen Auswahlbefehl sendet, um das Ein- und Ausschalten des fünften Schaltertransistors und des sechsten Schaltertransistors zu steuern.

4. Betriebssystem nach Anspruch 1 oder 2, **dadurch gekennzeichnet, dass**:
eine erste Ausgangsbuchse (21) der Schaltmatrix (4) mit einem Ende des ersten Ausführungsinstruments verbunden ist, und wobei eine zweite Ausgangsbuchse (22) der Schaltmatrix (4) jeweils mit einem Ende des zweiten Ausführungsinstruments und einem Ende der Schleifenelektrode (5) verbunden ist, wobei das erste Ausführungsinstrument ein bipolares Instrument (7) und das zweite Ausführungsinstrument eine Elektrodenstruktur (6) ist.

5. Betriebssystem nach Anspruch 4, **dadurch gekennzeichnet, dass**:
das bipolare Instrument (7) einen ersten leitfähigen Bereich (73), einen zweiten leitfähigen Bereich (74), eine Handgriffanordnung (75) sowie ein bipolares Instrument-Ausführungsende umfasst, wobei der erste leitfähige Bereich (73) mit einem ersten Ausgang der ersten Ausgangsbuchse (21) verbunden ist und der zweite leitfähige Bereich (74) mit einem zweiten Ausgang der ersten Ausgangsbuchse (21) verbunden ist, wobei die Handgriffanordnung (75) mit dem bipolaren Instrument-Ausführungsende verbunden ist, wobei das bipolare Instrument-Ausführungsende ein erstes Ausführungsende (71), ein zweites Ausführungsende (72) aufweist, und wobei die Elektrodenstruktur (6) ein drittes Ausführungsende aufweist.

## Revendications

1. Système d'opération chirurgicale, **caractérisé en ce qu'**il comprend
une unité de contrôle principale, un panneau de contrôle (1), un premier effecteur, un deuxième effecteur et une électrode de retour (5), ladite unité de contrôle principale comprend un contrôleur central (2), un module de source d'alimentation haute fréquence (3) et une matrice de commutation (4), ledit module de source d'alimentation haute fréquence (3) est connecté respectivement audit contrôleur central (2) et à ladite matrice de commutation (4), ledit module de source d'alimentation haute fréquence possède un mode de sortie monopolaire, un mode de sortie bipolaire, et un mode de sortie mixte monopolaire-bipolaire,
ledit contrôleur central (2) reçoit les instructions de sélection émises par ledit panneau de contrôle (1), ainsi que les informations de mode ou les instructions de pédale intégrées dans la puce de l'effecteur, ledit contrôleur central (2) émet des signaux de commande de commutation vers ladite matrice de commutation (4), afin de contrôler la conduction de ladite matrice de commutation (4) pour piloter ledit premier effecteur, ledit deuxième effecteur et ladite électrode de retour (5) pour fonctionner dans l'un des modes,
grâce à ladite unité de contrôle principale, ledit système d'opération chirurgicale fonctionne dans ledit mode de sortie monopolaire, ledit mode de sortie bipolaire, et ledit mode de sortie mixte monopolaire-bipolaire,
lorsque l'instruction de sélection dudit mode mixte monopolaire-bipolaire est obtenue, le contrôle dudit premier effecteur permet d'exécuter l'action de coupe ainsi que l'action de coagulation des tissus,
dans ledit mode mixte monopolaire-bipolaire, lors de l'exécution de l'action de coupe, ladite unité de contrôle principale, à l'aide d'un premier ensemble de signaux de contrôle, fait passer le courant à travers la première extrémité d'exécution dudit premier effecteur, traversant les tissus corporels pour atteindre l'électrode de retour, formant ainsi un premier circuit de courant, et réalise l'action de coupe en mode mixte monopolaire-bipolaire sur ledit premier effecteur,
dans ledit mode mixte monopolaire-bipolaire, lors de l'exécution de l'action de coagulation des tissus, ladite unité de contrôle principale, à l'aide d'un deuxième ensemble de signaux de contrôle, fait passer le courant à travers la première extrémité d'exécution dudit premier effecteur, traversant les tissus à exciser pour atteindre la deuxième extrémité d'exécution dudit premier effecteur, formant ainsi un deuxième circuit de courant, et réalise l'action de coagulation des tissus en mode mixte monopolaire-bipolaire sur ledit premier effecteur,
dans ledit mode monopolaire, ladite unité de contrôle principale, à l'aide d'un troisième ensemble de signaux de contrôle, fait passer le courant à travers la troisième extrémité d'exécution dudit deuxième effecteur, traversant les tissus corporels pour atteindre l'électrode de retour, formant ainsi un troisième circuit de courant, et réalise l'action de coupe ainsi que l'action de coagulation des tissus en mode monopolaire sur ledit deuxième effecteur,
dans ledit mode bipolaire, ladite unité de contrôle principale, à l'aide d'un quatrième ensemble de signaux de contrôle, fait passer le courant à travers la première extrémité d'exécution dudit premier effecteur, traversant les tissus corporels pour atteindre la deuxième extrémité d'exécution dudit premier effecteur, formant ainsi un quatrième circuit de courant, et réalise l'action de coupe ainsi que l'action de coagulation des tissus en mode bipolaire sur ledit premier effecteur.

2. Système d'opération selon la revendication 1, **caractérisé en ce que** :
ladite matrice de commutation (4) comprend un premier transistor (41), un deuxième transistor (42), un troisième transistor (43), un quatrième transistor (44), ledit contrôleur central (2) envoi des signaux de commande de commutation audit premier transistor (41), audit deuxième transistor (42), audit troisième transistor (43), audit quatrième transistor (44) en fonction des instructions de sélection reçues, afin de contrôler la conduction et la coupure dudit premier transistor (41), dudit deuxième transistor (42), dudit troisième transistor (43), dudit quatrième transistor (44).

3. Système d'opération selon la revendication 2, **caractérisé en ce que** :
ladite matrice de commutation (4) comprend également un cinquième transistor et un sixième transistor, ledit contrôleur central (2) envoi des signaux de commande de commutation audit cinquième transistor, audit sixième transistor en fonction des instructions de sélection reçues, afin de contrôler la conduction et la coupure dudit cinquième transistor, dudit septième transistor.

4. Système d'opération selon la revendication 1 ou 2, **caractérisé en ce que** :
la première prise de sortie (21) de ladite matrice de commutation (4) est connectée à l'extrémité dudit premier effecteur, la deuxième prise de sortie (22) de ladite matrice de commutation (4) est connectée respectivement à l'extrémité dudit deuxième effecteur et à l'extrémité de ladite électrode de retour (5), où ledit premier effecteur est un instrument bipolaire (7), ledit deuxième effecteur est une structure d'électrode (6).

5. Système d'opération selon la revendication 4, **caractérisé en ce que** :
ledit instrument bipolaire (7) comprend une première zone conductrice (73), une deuxième zone conductrice (74), un composant de poignée (75) ainsi qu'une extrémité d'exécution de l'instrument bipolaire, ladite première zone conductrice (73) est connectée à la première extrémité de sortie de ladite première prise de sortie (21), ladite deuxième zone conductrice (74) est connectée à la deuxième extrémité de sortie de ladite première prise de sortie (21), ledit composant de poignée (75) est connecté à ladite extrémité d'exécution de l'instrument bipolaire, où ladite extrémité d'exécution de l'instrument bipolaire possède une première extrémité d'exécution (71), une deuxième extrémité d'exécution (72), ladite structure d'électrode (6) possède une troisième extrémité d'exécution.
